# EUROPEAN PATENT APPLICATION

(11) **EP 4 793 294 A1**
(43) Date of publication of application: **19.08.2026**
(21) Application number: 25157652.6
(22) Date of filing: 13.02.2025
(51) Int. Cl.: C08C 19/02, C08C 19/04, C08F 136/06

(54) **TRANSESTERIFICATION PROCESS FOR MACROALCOHOLS**

(71) Applicant: Evonik Operations GmbH, 45128 Essen (DE)
(72) Inventor: ELSNER, Jens, 65239 Hochheim (DE); JANßEN, Dieter, 64823 Groß-Umstadt (DE); EISENBERG, Boris, 64646 Heppenheim (DE)
(74) Representative: Evonik Patent Association

(57) **Abstract**

The invention is directed to a process for preparing high molecular weight (meth)acrylic acid esters by transesterification of C1-4 alkyl (meth)acrylates with macroalcohols.

## Description

The invention is directed to a process for preparing high molecular weight (meth)acrylic acid esters by transesterification of C1-4 alkyl (meth)acrylates with macroalcohols.

(Meth)acrylic acid esters are widely known and frequently used monomers. Accordingly, various methods for obtaining these compounds are known. To prepare specialty (meth)acrylates, transesterification reactions are frequently used, wherein lower chain alkyl (meth)acrylates, like e.g. methyl (meth)acrylate, are reacted with an appropriate alcohol. To improve the yield and the selectivity of the reaction, different catalysts have been used to date.

Alkyl (meth)acrylates may be generally prepared by different methods: by reaction of acetone cyanohydrin with alcohols in the presence of sulphuric acid or by transesterification of methyl (meth)acrylate with alcohols in the presence of a catalyst. In the transesterification, both homogeneous and heterogeneous catalysts are used. Frequently, the reaction is carried out in the presence of a tetraalkyl titanate (tetraalkoxytitanium). The methanol released in the reaction is removed from the reaction mixture with the aid of a distillation column to positively influence the equilibrium of the transesterification reaction. The catalyst is removed by the addition of water and / or subsequent filtration and can be combined with a distillation / degassing step for further purification.

### State of the art

The literature discloses many transesterification processes carried out batchwise in conjunction with different catalysts.

When titanates are used as catalysts, the challenge is to proceed the reaction without the presence of water. That means that especially the used alcohols have to be dewatered before use as the presence of water during the reaction can have effects ranging from more significant deactivation of the tetraalkyl titanate used as a consequence of hydrolysis to the formation of undesired solid precipitates. Continuously operated processes (see e.g. US 6,977,310) often have the disadvantage that the catalyst is thermally stressed at relatively high temperatures in the liquid phase during rectification. This can easily lead to decomposition of the catalyst.

DE 2805702 discloses a method for preparing esters of unsaturated carboxylic acids by an ester exchange process which comprises reacting a lower alkyl ester of an unsaturated, 3-4 carbon atom carboxylic acid with a higher alcohol over a catalyst selected from the group consisting of chelate compounds of zirconium or calcium with beta-diketone or mixtures thereof. The reactions lead to high yields of about 97%, based on the alcohol used. The transesterification with a macroalcohol and the use of alkali methanolates as catalysts is not disclosed.

US 5,610,313 discloses a process for the preparation of alkyl imidazolidone (meth)acrylates that employs, as a catalyst, a mixture of at least one magnesium alkoxide and a component chosen from the chelates of calcium with 1 ,3-dicarbonyl compounds. Absolutely necessary is that a maximum dehydration is achieved before addition of the catalyst to the reactants. The transesterification with a macroalcohol and the use of alkali methanolates as catalysts is not disclosed.

In addition, it is possible to use acids or bases in order to catalyze the transesterification. Such reactions are disclosed, for example, in CN 1355161, DE 3423443 or EP 0534666. The basic catalysts include especially lithium amide, as detailed, by way of example, in publications DE 34 23 443, CA 795814 and US 6,194,530. The transesterification with a macroalcohol and the use of alkali methanolates as catalysts is not disclosed.

JP 2006-045284 describes a method for producing a photocurable oligomer chain-terminated (meth)acrylates, comprising subjecting a (meth)acrylic acid lower alkyl ester and an oligomer chain-terminated alcohol to an ester exchange reaction in the presence of an ester exchange catalyst, and then treating the resulting reaction mixture with a solid adsorbent in the presence of a mixed solvent comprising a hydrophilic solvent and a lipophilic solvent. A solvent-free transesterification reaction with a macroalcohol and the use of alkali methanolates as catalyst is not disclosed.

The reactions detailed above already lead to a high conversion and to pure products. Owing to the high economic significance of specialty (meth)acrylates, however, there is a permanent drive to further improve the preparation of these compounds, especially when specific starting materials are used.

It was an object of the present invention to find a process for the transesterification of specific macroalcohols which leads to high yields and high purities within short reaction times and avoids the disadvantages of working in the presence of a solvent and using water to remove the catalyst.

It was surprisingly found that by using the process according to the present invention, more than 95% up to 100% of the used macroalcohol can be converted into the corresponding ester. The process can preferably be carried out without the use of any additional solvent, i.e. there is no additional step to remove a solvent after the transesterification is finalized. Furthermore, the used catalyst can be easily removed by filtration, and a pure product is received which is not contaminated with any water.

For handling reasons, the higher molecular weight macroalcohols (e.g. Mₙ = 4,000 g/mol to 8,000 g/mol) can be used as solution in a base oil whereas the low or medium molecular weight macroalcohols (e.g. Mₙ = 800 g/mol to 2,000 g/mol) can be used as 100% without further dilution.

### Detailed description of the invention

The present invention is directed to a process for preparing high molecular weight (meth)acrylic acid esters by transesterification of C1-4 alkyl (meth)acrylates with macroalcohols in the presence of a catalyst.

The term "high molecular weight (meth)acrylic acid esters" refers to an ester of (meth)acrylic acid and a macroalcohol. The corresponding esters of (meth)acrylic acid with macroalcohols can also be referred to as macromonomers in the context of this invention.

The term "(meth)acrylic acid ester(s)" refers to both, ester(s) of acrylic acid and ester(s) of methacrylic acid. Esters of methacrylic acid are used preferably in accordance with the present invention.

The term "C1-4 alkyl (meth)acrylate(s)" refers to ester(s) of (meth)acrylic acid wherein the number of carbon atoms in the ester moiety is 1 to 4, preferably 1.

Specific examples of such low-chain alkyl esters include methyl (meth)acrylate, ethyl (meth)acrylate, n-propyl (meth)acrylate, isopropyl (meth)acrylate, n-butyl (meth)acrylate, isobutyl (meth)acrylate and tert-butyl (meth)acrylate. In accordance with the present invention, methyl (meth)acrylate and especially methyl methacrylate are preferably used.

In accordance with the present invention, macroalcohols are understood to be hydroxylated polyolefins having a high number-average molecular weight Mₙ. They can inter alia be prepared by living anionic polymerization of olefin monomers and subsequent functionalization with hydroxyl groups.

Living anionic polymerization is a well-known method for the polymerization of vinyl group containing monomers using anionic initiators [Baskaran, D. and Müller, A. H. E. (2009) Anionic Vinyl Polymerization, in Controlled and Living Polymerizations: From Mechanisms to Applications (eds A. H. E. Müller and K. Matyjaszewski), Wiley-VCH Verlag GmbH & Co. KGaA, Weinheim, Germany]. The term "living" refers to the fact that the polymerization generates polymer chains with stable carbanionic chain ends that retain their reactivity for a sufficient amount of time, thus enabling continued propagation without termination and transfer reactions. Because of the absence of termination and transfer reactions, each initiator molecule produces one living polymer chain. If the initiator is sufficiently reactive, the rate of initiation will be faster than the rate of chain propagation, and as a consequence, polymers with a narrow molecular weight distribution can be obtained. Due to the stability of the carbanionic chain and, the polymer chains can be functionalized with a diverse array of functional end groups by reaction with a variety of electrophilic reagents. Living anionic polymerization thus allows for the synthesis of well-defined, chain-end functionalized polymers with low degrees of compositional heterogeneity.

The synthesis of polybutadiene by living anionic polymerization of 1,3-butadiene using n-butyllithium as initiator is, for example, described in WO 2014/075901 or WO 2017/097702. By reaction of the carbanionic polybutadiene chain end with an epoxide such as propylene oxide, the polymer can be functionalized with a single hydroxyl group, yielding a mono-hydroxylated polybutadiene, which is of particular industrial importance.

For some applications, the resulting mono hydroxylated polybutadiene can further be hydrogenated to produce a fully saturated, long chain monoalcohol. A process for the preparation of a fully saturated monohydroxy-polybutadiene is, for example, described in WO 2015/040095 A1.

In accordance with the present invention, the term "macroalcohols" refers to alcohols, especially primary alcohols, having 30 to 600 carbon atoms in the alkyl chain. The alkyl chain can be straight chained or branched. Macroalcohols are preferably based on hydroxylated hydrogenated polybutadienes which have a number-average molecular weight Mₙ of 800 to 8,000 g/mol.

The number-average molecular weight Mₙ is determined by size exclusion chromatography using commercially available polybutadiene standards. The determination is affected according to DIN EN ISO 13885-1 by gel permeation chromatography with THF as eluent.

Preferably, the hydroxylated hydrogenated polybutadienes have a hydrogenation level of at least 95%, preferably at least 97%, preferably at least 99%, determined by NMR measurements.

Preferred hydroxylated hydrogenated polybutadienes can be obtained according to GB 2270317.

As used herein, the term "hydroxylated hydrogenated polybutadiene" refers to a hydrogenated polybutadiene that comprises one or more hydroxyl group. The hydroxylated hydrogenated polybutadiene may further comprise additional structural units, such as polyether groups derived from the addition of alkylene oxides to a polybutadiene or a maleic anhydride group derived from the addition of maleic anhydride to a polybutadiene. These additional structural units may be introduced into the polybutadiene when the polybutadiene is functionalized with hydroxyl groups.

Preference is given to monohydroxylated hydrogenated polybutadienes. More preferably, the hydroxylated hydrogenated polybutadiene used in accordance with the present invention is a hydroxyethyl- or hydroxypropyl-terminated hydrogenated polybutadiene. Particular preference is given to hydroxypropyl-terminated polybutadienes.

These monohydroxylated hydrogenated polybutadienes can be prepared by first converting butadiene monomers by anionic polymerization to polybutadiene. Subsequently, by reaction of the polybutadiene monomers with an alkylene oxide, such as ethylene oxide or propylene oxide, a hydroxy-functionalized polybutadiene can be prepared. The polybutadiene may also be reacted with more than one alkylene oxide units, resulting in a polyether-polybutadiene block copolymer having a terminal hydroxyl group. The hydroxylated polybutadiene can be hydrogenated in the presence of a suitable transition metal catalyst.

These monohydroxylated hydrogenated polybutadienes can also be selected from products obtained by hydroboration of (co)polymers of having a terminal double bond (e.g. as described in US Patent No. 4,316,973); maleic anhydride-ene-amino alcohol adducts obtained by an ene-reaction between a (co)polymer having a terminal double bond and maleic anhydride with an amino alcohol; and products obtained by hydroformylation of a (co)polymer having a terminal double bond, followed by hydrogenation (e.g. as described in JP Publication No. S63-175096).

The molar ratio of the low-chain (meth)acrylic acid ester: macroalcohol in the reaction mixture of the process is between 5:1 and 50:1, preferably between 5:1 and 30:1.

The mixture should be free of residual water before adding the catalyst.

The transesterification catalyst can be selected from the group consisting of tetraisopropyltitanate, ethylhexyltitanate, tetralauryltitanate, lithium methoxide, sodium methoxide, calcium hydroxide, magnesium hydroxide and mixtures thereof. According to the invention, the preferred catalyst is lithium methoxide either used in solid form or dissolved in methyl methacrylate or methanol as 10% solution. The catalyst is used in an amount of 0.01 wt% to 0.8 wt%, preferably 0.1 wt% to 0.3 wt%, based on the weight of the macroalcohol used, or alternatively 0.01 molar-% to 0.8 molar-%, preferably 0.05 molar-% to 0.35 molar-%, based on the amount of macroalcohol used.

The transesterification is an equilibrium reaction wherein a low molecular weight alcohol is released. To shift the equilibrium to the product side, the low molecular weight alcohol is typically removed from the reaction system, for example by distillation.

The transesterification is carried out in a suitable reaction vessel which can be either a single reaction tank or also a battery of two or more reaction tanks connected in series. Each reaction vessel is equipped with a stirrer and at least one reflux condenser and should have a vapour outlet to the azeotrope distillation column for removing the low molecular weight alcohol released in the reaction.

To perform the reaction, the C1-4 alkyl (meth)acrylate and the macroalcohol and, if necessary, one or more polymerization inhibitor(s) or stabilizer(s) are charged into the reaction vessel.

Optionally, suitable solvents can be used. Examples of solvents usable in an ester exchange reaction include organic solvents such as aliphatic hydrocarbon compounds such as n-hexane and heptane; cycloaliphatic hydrocarbon compounds such as cyclohexane; aromatic hydrocarbon compounds such as toluene; and ether compounds such as tetrahydrofuran, ethylene glycol dimethyl ether and ethylene glycol diethyl ether; ketones such as acetone and mixtures thereof.

In accordance with the present invention, the process is preferably carried out in the absence of any additional solvent.

Several alcohols are suitable as the reactant alcohol in the process of the invention and include, for example, without limitation aliphatic linear and branched chain alcohols. Preferred alcohols are characterized in that a macroalcohol is used with a weight-average molecular weight Mₙ from 800 g/mol to 8000 g/mol, preferably from 1000 g/mol to 5000 g/mol, preferably from 2000 g/mol to 4800 g/mol.

The macroalcohol contains preferably one hydroxyl group; the content of a secondary hydroxyl-functionality is equal or less than 0.1%.

These macroalcohols may be used singly or in combination of two or more different ones.

In a preferred embodiment, the macroalcohols are monohydroxylated hydrogenated polybutadienes.

The reaction mixture may further comprise one or more polymerization inhibitors or stabilizers.

The polymerization inhibitor/stabilizer is selected from the group consisting of hydrochinone monomethylether, 4-hydroxy-2,2,6,6-tetramethylpiperidinooxyl (TMP), octadecyl-3-(3,5-di-tert-butyl-4-hydroxyphenyl)-propionat, 4-methyl-2,6-di-tert-butylphenyl and mixtures thereof. Preference is given to hydrochinone monomethylether, 4-hydroxy-2,2,6,6-tetramethylpiperidinooxyl (TMP) and mixtures thereof.

A permanent air stream of 5 L/h to 80 L/h (corresponding to 6 vol% to 21 vol% of oxygen) through the reaction mixture is maintained in order ensure activity of the inhibitor.

The pressure, at which the transesterification is carried out, is not particularly limited. The transesterification can be performed at ambient pressure, reduced pressure or elevated pressure.

The transesterification is preferably carried out at a temperature between 70°C and 155°C, more preferably at the boiling point of the used C1-4 alkyl (meth)acrylate. In case of methyl methacrylate, the temperature is between 95°C and 100°C.

It is well known in the art that a lower pressure also lowers the boiling point.

To ensure a proper heating of the reaction mixture, the reaction vessel is preferably surrounded by a medium which can rapidly heat and efficiently cool down the reaction mixture.

According to the invention, the entire amount of the catalyst is added bevor the first reaction step. In a preferred embodiment, 2/3 of the total amount of the catalyst is added bevor the first reaction step and 1/3 of the total amount is added after a period of time, when the release rate of the by-produced lower alcohol decreases.

During the reaction, the by-produced C1-4 alkyl (meth)acrylate/C1-4 alkyl alcohol azeotrope can be removed from the reaction system by batch or continuous distillation.

For removing the catalyst contained in the reaction mixture and to therefore improve the purity of the targeted macromonomer, commonly used filtration aids can be used to which the catalyst is adsorbed. Preferred filtration aids are, for example, functional cellulose fibers like the commercially available Arbocel^{®} FIC 200 (JRS, Germany), Arbocel^{®} NV00 (JRS, Germany) or calcined diatomaceous earth like the commercially available Dicalite^{®} (Dicalite, Germany).

After the adsorption treatment is thus performed, the solid filtration aid and impurities contained in the mixture are removed by filtration.

For the filtration, filtration under standard pressure, pressure filtration or filtration under reduced pressure, centrifugal filtration or the like can be employed.

The amount of filtration aid to be used in accordance with the present invention is preferably 0.1 wt% to 3 wt%, more preferably 0.5 wt% to 1.5 wt%, based on the total weight of the resulting esters of (meth)acrylic acid with macroalcohols.

In the ester exchange reaction, a lower alcohol is by-produced as the ester exchange reaction proceeds. Preferably, the by-produced lower alcohol is removed outside the reaction system as an azeotropic mixture with the (meth)acrylic acid lower alkyl ester or the reaction solvent. Various possibilities for batch distillation of azeotropic mixtures are common general knowledge.

As the starting material is a C1-4 alkyl (meth)acrylate, the corresponding C1-4 alkyl alcohol is prepared during the reaction. In accordance with the definition of the term "C1-4 alkyl (meth)acrylate(s)" given further above, such C1-4 alkyl alcohol can be methanol, ethanol, n-propanol, isopropanol, n-butanol, isobutanol and tert-butanol. In accordance with the present invention, methanol is preferably produced.

The present invention is further directed to a process for preparing a high molecular weight (meth)acrylic acid ester by transesterification of a C1-4 alkyl (meth)acrylate with a macroalcohol, comprising the steps of:
(a) charging a reaction vessel equipped with stirrer and reflux condenser with a C1-4 alkyl (meth)acrylate and a macroalcohol in a ratio of between 5:1 and 50:1 and at least one stabilizer;
(b) maintaining a permanent air stream of 5 L/h to 80 L/h (corresponding to 6 vol% to 21 vol% of oxygen) through the reaction mixture;
(c) optionally, heating the reaction mixture obtained under (b) until a stable reflux is reached and distilling about 1% to 10% of the C1-4 alkyl (meth)acrylate to remove water from the reaction system;
(d) adding 0.1 wt% to 0.3 wt% of a catalyst, based on the total amount of macroalcohol used, in solid form or as a 10% solution in methyl methacrylate or methanol;
(e) heating the reaction mixture obtained under (d) until the head temperature of the condenser reaches reflux temperature of the lower chain alkyl (meth)acrylate used or its azeotrope with the released low molecular alcohol;
(f1) constantly distilling an azeotropic mixture of the C1-4 alkyl (meth)acrylate and the corresponding C1-4 alkyl alcohol which is built during transesterification until the head temperature of the condenser reaches the temperature of the pure C1-4 alkyl (meth)acrylate used; or
(f2) holding the reaction mixture obtained under (e) under total reflux to enrich the corresponding C1-4 alkyl alcohol which is built during transesterification in the azeotrope and taking off the azeotrope in certain intervals;
(g) after the corresponding C1-4 alkyl alcohol is completely removed, removing of the excessive C1-4 alkyl (meth)acrylate under reduced pressure during a time of 1 to 2 hours;
(h) filling the reaction vessel with air or nitrogen to ambient pressure and cooling down the reaction mixture obtained under (g) to a temperature in the range of 80°C to 100°C;
(i) adding 0.1 wt% to 3 wt% of a filtration aid, based on the total weight of the high molecular weight (meth)acrylic acid ester, and mixing; and
(j) cooling down the mixture obtained under (i) to a temperature of about 70°C and filtrating it by using a filter to receive the high molecular weight (meth)acrylic acid ester.

The reaction vessel used in step (a) can be equipped with an external heating jacket, external heating coils, internal heating coils, an external circulation loop including a heat exchanger or a combination of any of the aforementioned.

The stabilizer used in step (a) can be selected from the group consisting of hydrochinone monomethylether, 4-hydroxy-2,2,6,6-tetramethylpiperidinooxyl (TMP), octadecyl-3-(3,5-di-tert-butyl-4-hydroxyphenyl)-propionat, 4-methyl-2,6-di-tert-butylphenyl and mixtures thereof. Preference is given to hydrochinone monomethylether, 4-hydroxy-2,2,6,6-tetramethylpiperidinooxyl (TMP) and mixtures thereof. In addition, a 2nd stabilizer can be introduced solely or in combination with the 1st one as process stabilizer through the distillation column.

Step (c) is optionally used in cases where the ratio of C1-4 alkyl (meth)acrylate and macroalcohol exceeds 10:1 to remove residual water that may be added with the alkyl (meth)acrylate.

The catalyst used in step (d) can be selected from the group consisting of tetraisopropyltitanate, ethylhexyltitanate, tetralauryltitanate, lithium methoxide, sodium methoxide, calcium hydroxide, magnesium hydroxide and mixtures thereof. According to the invention, the preferred catalyst is lithium methoxide either used in solid form or dissolved in methyl methacrylate or methanol as 10% solution. The catalyst is used in an amount of 0.01 wt% to 0.8 wt%, preferably 0.1 wt% to 0.3 wt%, based on the weight of the macroalcohol used, or alternatively 0.01 molar-% to 0.8 molar-%, preferably 0.05 molar-% to 0.35 molar-%, based on the amount of macroalcohol used.

In step (f1) and/or (f2), the loss of C1-4 alkyl (meth)acrylate due to offtake of the low boiling azeotrope mixture can be optionally balanced by adding the correspondent volume of C1-4 alkyl (meth)acrylate over time or at once during the reaction phase

The filtration aid used in step (i) can be selected from the group consisting of functional cellulose fibers like the commercially available Arbocel^{®} FIC 200 (JRS, Germany), Arbocel^{®} NV00 (JRS, Germany) and calcined diatomaceous earth like the commercially available Dicalite^{®} (Dicalite, Germany). It can be added directly to the reaction vessel or in a separate precipitation vessel equipped with a stirrer and an optional circulation loop for introducing and homogenizing the filtration aid into the reaction mixture.

The present invention is further directed to a process for preparing a high molecular weight (meth)acrylic acid ester by transesterification of methyl (meth)acrylate with a macroalcohol, comprising the steps of:
(a) charging a reaction vessel equipped with stirrer and reflux condenser with methyl methacrylate and a macroalcohol in a ratio of between 5:1 and 30:1, the macroalcohol having a number-average molecular weight Mₙ of 1000 g/mol to 5000 g/mol, and at least one stabilizer which is selected from the group consisting of hydrochinone monomethylether, 4-hydroxy-2,2,6,6-tetramethylpiperidinooxyl, octadecyl-3-(3,5-di-tert-butyl-4-hydroxyphenyl)-propionate, 4-methyl-2,6-di-tert-butylphenyl and mixtures thereof;
(b) maintaining a permanent air stream of 5 L/h to 80 L/h (corresponding to 6 vol% to 21 vol% of oxygen) through the reaction mixture;
(c) optionally, heating the reaction mixture obtained under (b) until a stable reflux is reached and distilling about 1% to 10% of the methyl methacrylate to remove water from the reaction system;
(d) adding 0.1 wt% to 0.3 wt% of lithium methoxide, based on the total weight of the reaction mixture, as solid catalyst;
(e) heating the reaction mixture obtained under (d) until the head temperature of the condenser reaches reflux temperature of 65°C to 100°C;
(f1) constantly distilling off an azeotropic mixture of methyl methacrylate and methanol which is built during transesterification until the head temperature of the condenser reaches a temperature of about 95°C to 100°C; or
(f2) holding the reaction mixture obtained under (e) under total reflux to enrich the methanol which is built during transesterification in the azeotrope and taking off the azeotrope from the head of the distillation column after certain intervals;
(g) after methanol is completely removed, removing the excessive methyl methacrylate under reduced pressure during a time of 1 to 2 hours;
(h) filling the reaction vessel with air or nitrogen to ambient pressure and cooling down the reaction mixture obtained under (g) to a temperature of about 80°C to 100°C;
(i) adding 0.1 wt% to 3 wt% of a filtration aid, based on the total weight of the high molecular weight (meth)acrylic acid ester, and mixing; and
(j) cooling down the mixture obtained under (i) to a temperature of about 70°C and filtrating it by using a filter to receive the macromonomer.

In step (a), additionally a stream of a solution of a 2^{nd} stabilizer in methyl methacrylate can be introduced as process stabilizer through the distillation column.

The macromonomers obtained by the process of the present invention can be used for the preparation of polymers or copolymers.

These polymers or copolymers can be used as additives in lubricant compositions, e.g. as viscosity index improver, antioxidants, antiwear agents, dispersants, detergents, friction modifiers, antifoam agents, extreme pressure additives, corrosion inhibitors and pour point depressants.

These polymers or copolymers and the lubricant compositions comprising them are favorably used for driving system lubricating oils (such as manual transmission fluids, differential gear oils, automatic transmission fluids and belt-continuously variable transmission fluids, axle fluid formulations, dual clutch transmission fluids, and dedicated hybrid transmission fluids), hydraulic oils (such as hydraulic oils for machinery, power steering oils, shock absorber oils), engine oils (for gasoline engines and for diesel engines), industrial oil formulations (such as wind turbines) and fluids for thermal management (such as datacenter or battery cooling).

The invention will be illustrated in detail below by examples and comparative examples without any intention that the concept of the invention be restricted to these particular embodiments.

### Experimental Part

### Abbreviations

- Arbocel^{®}: functional cellulose product, commercially available from JRS, Germany
- LiOMe: lithium methoxide
- MA-1: macroalcohol of hydrogenated polybutadiene (Mₙ = 2,000 g/mol)
- MA-2: macroalcohol of hydrogenated polybutadiene (Mₙ = 4,500 g/mol)
- MeOH: methanol
- MM-1: macromonomer of hydrogenated polybutadiene with methacrylate functionality (Mₙ = 2,000 g/mol)
- MM-2: macromonomer of hydrogenated polybutadiene with methacrylate functionality (Mₙ = 4,500 g/mol)
- MMA: methyl methacrylate
- Mₙ: number-average molecular weight
- M_{w}: weight-average molecular weight
- NB3020: Nexbase^{®} 3020, Group III base oil from Neste with a KV100 of 2.2 mm²/s and a KV40 of 7.73 mm²/s
- THF: tetrahydrofuran
- Tempol: 4-hydroxy-2,2,6,6-tetramethylpiperinooxyl

### Test Methods

The number-average molecular weight Mₙ of the macromonomers is determined by size exclusion chromatography using commercially available polybutadiene standards. The determination is effected to DIN EN ISO 13885-1 by gel permeation chromatography with THF as eluent.

Molecular weights of the macromonomers were determined by size exclusion chromatography (SEC) using commercially available polypropylene glycol (PPG, 76 to 6000 g/mol) standards. The determination is effected by gel permeation chromatography with THF as eluent (flow rate: 1 mL/min; injected volume: 100 µL).

| | | | | |
|---|---|---|---|---|
| Columns: | 5 SDV Columns 8 x 300 mm resp. 8 x 50 mm (by PSS at Mainz) | | | |

| | No. | Type | | Dimension |
|---|---|---|---|---|
| | Precolumn | SDV | 10 µ | 8 X 50 mm |
| | 1 | SDV LinXL | 10 µ | 8 X 300 mm |
| | 2 | SDV LinL | 10 µ | 8 X 300 mm |
| | 3 | SDV 100 Å | 10 µ | 8 X 300 mm |
| | 4 | SDV 100 Å | 10 µ | 8 X 300 mm |
| | 5 | KF-800D | 5 µ | 8 X 100 mm |
| Instruments: | Isocratic HPLC System with degasser, autosampler, column heater (oven), RI / UV detector according to the requirements of the ISO standard | | | |
| Oven: | Temperature 35°C | | | |
| Eluent: | Tetrahydrofuran | | | |
| | Eluent is continuously distilled and circulated by pump | | | |
| Flow rate: | 1 mL / min | | | |
| Injected volume: | 20 µL | | | |
| Detection: | Rl: Temperature 35°C | | | |
| | UV: wave langht 239 nm | | | |
| Software: | PSS WinGPC-Software | | | |
| Concentration sample solution: | 2 g/L (Mw > 106: 1 g/L ... 0.5 g/L) | | | |
| Standards: | PPG (for example PSS (Mainz)) | | | |
| Concentration standard solution: | 0.25% solutions | | | |

### Degree of hydrogenation

The degree of hydrogenation and the relative proportion of OH-functions in macroalcohols was determined using 1H-NMR spectroscopy. It can also determine the proportion of 1,2-adduct in polybutadiene. The method involves recording a 1H-NMR spectrum.

In principle, the method is applicable to all hydrogenated macroalcohols with a polybutadiene microstructure.

Approximately 100 mg of the sample is dissolved in about 1 mL of CDCI3. Once a clear solution is obtained, about 0.6 mL of the solution is transferred to an NMR tube and measured. If necessary, the temperature can be increased during the dissolving process.

### ¹H-NMR was recorded with the following parameters:

| | |
|---|---|
| Device: | e.g. Bruker Avance 400 |
| Nucleus: | ¹H |
| Solvent: | Chloroform-d (CDCl3) |
| Puls angle: | 30° |
| Relaxation delay: | 5 s |
| Number of scans: | minimum 256 |
| Reference signal: | TMS at 0,00 ppm |
| Temperature: | 323 K |

### Synthesis of macroalcohols (hydroxylated hydrogenated polybutadiene) MA-1 and MA-2

The macroalcohols were synthesized by anionic polymerization of 1 ,3-butadiene with butyllithium at 20 to 45°C. On attainment of the desired degree of polymerization, the reaction was stopped by adding propylene oxide and lithium was removed by precipitation with methanol. Subsequently, the polymer was hydrogenated under a hydrogen atmosphere in the presence of a noble metal catalyst (Pd-, Ru- or Pt-based) at up to 140°C and 200 bar pressure. After the hydrogenation had ended, the noble metal catalyst was removed and organic solvent was drawn off under reduced pressure. Finally, MA-2 was diluted with NB3020 to a macroalcohol content of 70 wt%, whereas MA-1 was kept 100 wt%. Table 1 summarizes the characterization data of MA-1 and MA-2.

**Table 1: Characterization data of macromonomers MA-1 and MA-2.**

| | **Mₙ [g/mol]** | **Hydrogenation level [%]** | **OH functionality [%]** |
|---|---|---|---|
| MA-1 | 2,000 | >99 | >98 |
| MA-2 | 4,500 | >99 | >98 |

### Synthesis of macromonomers MM-1

### Example 1: MMA:MA-1 = 15:1

A reaction vessel equipped with a stirrer, a thermometer, a reflux condenser and a gas introduction apparatus was charged with 700 g (0.35 mol) of macroalcohol MA-1 (100 wt%), 525 g (5.25 mol) of MMA, 0.0525 g (100 ppm, based on the amount of MMA used) of hydrochinone monomethylether, 0.026 g (50 ppm, based on the amount of MMA used) of Tempol.

After charging the raw materials and start of stirring and air bubbling with 20 L/h, the reaction mixture was heated to reflux and 100% distillate taken off until 20 mL distillate (corresponding to about 2% of the MMA as initially used) was collected for dewatering.

The resulting mixture was heated under reflux until the head temperature of distillation column was at 100°C again.

1.89 g (0.27 wt% or 0.14 mol%, based on the amount of MA-1 used, 1:10 dissolved in MeOH) of lithium methoxide was entered to the remaining reaction mixture.

In steps of 1 hour, the distillate was distilled off in portions to the outside of the reaction system until the temperature in the column head reached 100°C again every time. After 4 times distillate take off, the reaction mixture was stirred at 137°C under a vacuum of 15 mbar for completion of the reaction for 1-2 hours while excess MMA was completely distilled off.

After completion of the reaction, the reactor was filled with air to ambient pressure and the heating was stopped.

At a temperature of 100°C, 7 g of the filtration aid Arbocel^{®} FIC 200 (1 wt%, based on the total weight of MM-1) was added and the mixture stirred for 30 minutes at 70°C.

The resulting reaction mixture was filtered at a temperature of 70°C using a SEITZ-T 1000 filter.

The yield (based on the raw material alcohol) was 100%.

### Example 2: MMA:MA-1 = 15:1

A reaction vessel equipped with a stirrer, a thermometer, a reflux condenser and a gas introduction apparatus was charged with 1800 g (0.84 mol) of macroalcohol MA-1 (100%), 1200 g (12.6 mol) of MMA, 0.25 g (200 ppm, based on the amount of MMA used) of hydrochinone monomethylether, 0.06 g (50 ppm, based on the amount of MMA used) of Tempol.

After charging the raw materials and start of stirring and air bubbling with 20 L/h, the reaction mixture was heated to reflux until the head temperature of distillation column was at 100°C.

3.01 g (0.095 wt% or 0.17 mol%, based on the amount of MA-1 used, 1:10 dissolved in MeOH) of lithium methoxide was entered to the reaction mixture.

In steps of 1 hour, the distillate was distilled off in portions to the outside of the reaction system until temperature in the column head reached 100°C again every time. After 4 times distillate take off the reaction mixture was stirred at 139°C under vacuum of 20 mbar for completion of the reaction for 1-2 hours while excess MMA was completely distilled off.

After completion of the reaction, the reactor was filled with air to ambient pressure and the heating was stopped.

At a temperature of 100°C, 18 g of the filtration aid Arbocel^{®} FIC 200 (1 wt%, based on the total weight of MM-1) was added and the mixture stirred for 30 minutes at 70°C.

The resulting reaction mixture was filtered at a temperature of 70°C using a SEITZ-T 1000 filter.

The yield (based on the raw material alcohol) was ≥ 95%.

### Example 3: MMA:MA-1 = 15:1

A reaction vessel equipped with a stirrer, a thermometer, a reflux condenser and a gas introduction apparatus was charged with 1926 g (0.9 mol) of macroalcohol MA-1 (100%), 1347 g (13.5 mol) of MMA, 0.27 g (200 ppm, based on the amount of MMA used) of hydrochinone monomethylether, 0.06 g (50 ppm, based on the amount of MMA used) of Tempol.

After charging the raw materials and start of stirring and air bubbling with 20 L/h, the reaction mixture was heated to reflux until the head temperature of distillation column was at 100°C.

3.3 g (0.17 wt% or 0.095 mol%, based on the amount of MA-1 used, 1:10 dissolved in MeOH) of lithium methoxide was entered to the reaction mixture.

In steps of 1 hour, the distillate was distilled off in portions to the outside of the reaction system until temperature in the column head reached 100°C again every time. After 4 times distillate take off the reaction mixture was stirred at 138°C under vacuum of 12 mbar for completion of the reaction for 1-2 hours while excess MMA was completely distilled off.

After completion of the reaction, the reactor was filled with air to ambient pressure and the heating was stopped.

At a temperature of 100°C, 18 g of the filtration aid Arbocel^{®} FIC 200 (1 wt%, based on the total weight of MM-1) was added and the mixture stirred for 30 minutes at 70°C.

The resulting reaction mixture was filtered at a temperature of 70°C using a SEITZ-T 1000 filter.

The yield (based on the raw material alcohol) was ≥ 95%.

### Example 4: MMA:MA-1 = 10:1

A reaction vessel equipped with a stirrer, a thermometer, a reflux condenser and a gas introduction apparatus was charged with 3000 g (1.5 mol) of macroalcohol MA-1 (100%), 1500 g (15 mol) of MMA, 0.3 g (200 ppm, based on the amount of MMA used) of hydrochinone monomethylether, 0.075 g (50 ppm, based on the amount of MMA used) of Tempol.

After charging the raw materials and start of stirring and air bubbling with 20 L/h, the reaction mixture was heated to reflux until the head temperature of distillation column was at 100°C.

4.5 g (0.15 wt% or 0.08 mol%, based on the amount of MA-1 used, 1:10 dissolved in MeOH) of lithium methoxide was entered to the reaction mixture.

In steps of 1 hour, the distillate was distilled off in portions to the outside of the reaction system until temperature in the column head reached 100°C again every time. After 4 times distillate take off the reactor was filled with air to ambient pressure and the heating was stopped.

At a temperature of 100°C, 15 g of the filtration aid Arbocel^{®} FIC 200 (0.5 wt%, based on the total weight of MM-1) was added and the mixture stirred for 45 minutes at 70-80°C.

The resulting reaction mixture was filtered at a temperature of 70°C using a SEITZ-T 2600 filter. The reaction mixture was further heated up to 140°C and stirred under vacuum of 10 mbar for 1-2 hours to remove excess MMA completely.

The final reaction mixture was filtered again at a temperature of 70°C using a SEITZ-T 1000 filter.

The yield (based on the raw material alcohol) was 100%.

### Example 5: MMA:MA-1 = 6:1

A reaction vessel equipped with a stirrer, a thermometer, a reflux condenser and a gas introduction apparatus was charged with 3280 g (1.15 mol) of macroalcohol MA-1 (70% in NB3020), 690 g (6.9 mol) of MMA, 0.14 g (200 ppm, based on the amount of MMA used) of hydrochinone monomethylether, 0.04 g (50 ppm, based on the amount of MMA used) of Tempol.

After charging the raw materials and start of stirring and air bubbling with 20 L/h, the reaction mixture was heated to reflux until the head temperature of distillation column was at 100°C.

4 g (0.17 wt% or 0.087 mol%, based on the amount of MA-1 used, 1:10 dissolved in MeOH) of lithium methoxide was entered to the reaction mixture.

In steps of 1 hour, the distillate was distilled off in portions to the outside of the reaction system until temperature in the column head reached 100°C again every time. After 4 times distillate take off the reactor was filled with air to ambient pressure and the heating was stopped.

At a temperature of 90°C, 11.5 g of the filtration aid Arbocel^{®} FIC 200 (0.5 wt%, based on the total weight of MM-1) was added and the mixture stirred for 30 minutes at 90°C.

The resulting reaction mixture was filtered at a temperature of 70°C using a SEITZ-T 2600 filter. The reaction mixture was further heated up to 140°C and stirred under vacuum of 40 mbar for 1-2 hours to remove excess MMA completely.

The final reaction mixture was filtered again at a temperature of 70°C using a SEITZ-T K900 filter.

The yield (based on the raw material alcohol) was 99.5%.

### Example 6: MMA:MA-1 = 8:1

A reaction vessel equipped with a stirrer, a thermometer, a reflux condenser and a gas introduction apparatus was charged with 3429 g (1.2 mol) of macroalcohol MA-1 (70% in NB3020), 970 g (9.7 mol) of MMA, 0.14 g (200 ppm, based on the amount of MMA used) of hydrochinone monomethylether, 0.04 g (50 ppm, based on the amount of MMA used) of Tempol.

After charging the raw materials and start of stirring and air bubbling with 20 L/h, the reaction mixture was heated to reflux until the head temperature of distillation column was at 100°C.

4 g (0.17 wt% or 0.087 mol%, based on the amount of MA-1 used, 1:10 dissolved in MeOH) of lithium methoxide was entered to the reaction mixture.

In steps of 1 hour, the distillate was distilled off in portions to the outside of the reaction system until temperature in the column head reached 100°C again every time. After 4 times distillate take off the reactor was filled with air to ambient pressure and the heating was stopped.

At a temperature of 90°C, 11.5 g of the filtration aid Arbocel^{®} FIC 200 (0.5 wt%, based on the total weight of MM-1) was added and the mixture stirred for 30 minutes at 90°C.

The resulting reaction mixture was filtered at a temperature of 70°C using a SEITZ-T 2600 filter. The reaction mixture was further heated up to 135°C and stirred under vacuum of 10 mbar for 1-2 hours to remove excess MMA completely.

The final reaction mixture was filtered again at a temperature of 70°C using a SEITZ-T K900 filter.

The yield (based on the raw material alcohol) was 99.5%.

### Example 7: MMA:MA-1 = 5:1

A reaction vessel equipped with a stirrer, a thermometer, a reflux condenser and a gas introduction apparatus was charged with 4000 g (1.4 mol) of macroalcohol MA-1 (70% in NB3020), 700 g (7 mol) of MMA, 0.14 g (200 ppm, based on the amount of MMA used) of hydrochinone monomethylether, 0.04 g (50 ppm, based on the amount of MMA used) of Tempol.

After charging the raw materials and start of stirring and air bubbling with 20 L/h, the reaction mixture was heated to reflux until the head temperature of distillation column was at 100°C.

4.7 g (0.16 wt% or 0.085 mol%, based on the amount of MA-1 used, 1:10 dissolved in MeOH) of lithium methoxide was entered to the reaction mixture.

In steps of 1 hour, the distillate was distilled off in portions to the outside of the reaction system until temperature in the column head reached 100°C again every time. After 4 times distillate take off the reactor was filled with air to ambient pressure and the heating was stopped.

At a temperature of 90°C, 5.6 g of the filtration aid Arbocel^{®} FIC 200 (0.2 wt%, based on the total weight of MM-1) was added and the mixture stirred for 30 minutes at 90°C.

The resulting reaction mixture was filtered at a temperature of 70°C using a SEITZ-T 1000 filter. The reaction mixture was further heated up to 135°C and stirred under vacuum of 10 mbar for 1-2 hours to remove excess MMA completely.

The final reaction mixture was filtered again at a temperature of 70°C using a SEITZ-T T1000 filter.

The yield (based on the raw material alcohol) was 96.4%.

### Example 8: MMA:MA-1 = 8:1

A reaction vessel equipped with a stirrer, a thermometer, distillation column, a reflux condenser and a gas introduction apparatus was charged with 3775 kg (1887.5 mol) of macroalcohol MA-1 (100%), 1520 kg (15200 mol) of MMA, 400 g (100 ppm, based on the amount of MMA used) of hydrochinone monomethylether.

After charging the raw materials stirring and air bubbling with 8000 L/h were started as well as stabilizer dosing of 5 L/h (3000 ppm MEHQ and 50 ppm Tempol in MMA) into the column head. The reaction mixture was then heated to reflux and 100% distillate taken off until 93 L of distillate (corresponding to about 6% of the MMA as initially used) was collected for dewatering. 9 kg (0.24 wt% or 0.125 mol%, based on the amount of MA-1 used) of lithium methoxide was entered to the reaction mixture within 2 portions; 7kg after dewatering step and 2 kg after 4 hours of reaction time.

In steps of 1 hour, the distillate was distilled off in portions to the outside of the reaction system until temperature in the column head reached 100°C again every time. After 4 times distillate take off the reaction mixture was further heated up to 138°C and stirred under vacuum of 78mbar for 1 hour to remove excess MMA completely.

The reactor was filled with air to ambient pressure and the heating was stopped.

At a temperature of 90°C, 15 kg of the filtration aid Arbocel^{®} FIC 200 (0.2 wt%, based on the total weight of MM-1) was added and the mixture stirred for 120 minutes at 90-100°C.

The resulting reaction mixture was filtered at a temperature of 58°C using a rotating disc filter while in the first 15 min the material was circulated over the filter before switching to throughput filtration.

The yield (based on the raw material alcohol) was 99.8%.

### Synthesis of macromonomers MM-2

### Example 9: MMA:MA-2 = 22:1

A reaction vessel equipped with a stirrer, a thermometer, a reflux condenser and a gas introduction apparatus was charged with 964 g (0.15 mol) of macroalcohol MA-2 (70% in NB3020), 330 g (3.3 mol) of MMA, 0.048 g (100 ppm, based on the amount of MMA used) of hydrochinone monomethylether, 0.026 g (50 ppm, based on the amount of MMA used) of Tempol.

After charging the raw materials and start of stirring and air bubbling with 20 L/h, the reaction mixture was heated to reflux and 100% distillate taken off until 20 mL distillate (corresponding to about 2% of the MMA as initially used) was collected for dewatering.

The resulting mixture was heated under reflux until the head temperature of distillation column was at 100°C again.

1.82 g (0.27 wt% or 0.32 mol%, based on the amount of MA-2 used, 1:10 dissolved in MeOH) of lithium methoxide was entered to the remaining reaction mixture.

In steps of 1 hour, the distillate was distilled off in portions to the outside of the reaction system until temperature in the column head reached 100°C again every time. After 4 times distillate take off the reaction mixture was stirred at 130°C under vacuum of 15 mbar for completion of the reaction for 1-2 hours while excess MMA was completely distilled off.

After completion of the reaction, the reactor was filled with air to ambient pressure and the heating was stopped.

At a temperature of 100°C, 9.6 g of the filtration aid Arbocel^{®} FIC 200 (1 wt%, based on the total weight of MM-2) was added and the mixture stirred for 30 minutes until reaching 70°C.

The resulting reaction mixture was filtered at a temperature of 70°C using a SEITZ-T 1000 filter.

The yield (based on the raw material alcohol) was 100%.

### Example 10: MMA:MA-2 = 28.5:1

A reaction vessel equipped with a stirrer, a thermometer, a reflux condenser and a gas introduction apparatus was charged with 695 g (0.1 mol) of macroalcohol MA-2 (70% in NB3020), 285.7 g (15 mol) of MMA, 0.05 g (200 ppm, based on the amount of MMA used) of hydrochinone monomethylether, 0.01 g (50 ppm, based on the amount of MMA used) of Tempol.

After charging the raw materials and start of stirring and air bubbling with 20 L/h, the reaction mixture was heated to reflux until the head temperature of distillation column was at 100°C.

0.95 g (0.19 wt% or 0.25 mol%, based on the amount of MA-2 used, 1:10 dissolved in MeOH) of lithium methoxide was entered to the reaction mixture.

In steps of 1 hour, the distillate was distilled off in portions to the outside of the reaction system until temperature in the column head reached 100°C again every time. After 4 times distillate take off the reactor was filled with air to ambient pressure and the heating was stopped.

At a temperature of 100°C, 6.9 g of the filtration aid Arbocel^{®} FIC 200 (1 wt%, based on the total weight of MM-1) was added and the mixture stirred for 30 minutes until reaching 70°C.

The resulting reaction mixture was filtered at a temperature of 70°C using a SEITZ-T 1000 filter. The reaction mixture was further heated up to 120°C and stirred under vacuum of 12 mbar for 1-2 hours to remove excess MMA completely.

The final reaction mixture was filtered again at a temperature of 70°C using a SEITZ-T 1000 filter.

The yield (based on the raw material alcohol) was 100%.

### Example 11: MMA:MA-2 = 20.7 :1

A reaction vessel equipped with a stirrer, a thermometer, a reflux condenser and a gas introduction apparatus was charged with 1000 g (0.15 mol) of macroalcohol MA-2 (70% in NB3020), 156 g (1.5 mol) of MMA, 0.06 g (200 ppm, based on the amount of MMA used) of hydrochinone monomethylether, 0.016 g (50 ppm, based on the amount of MMA used) of Tempol.

After charging the raw materials and start of stirring and air bubbling with 20 L/h, the reaction mixture was heated to reflux and 100% distillate taken off until 10 mL distillate (corresponding to about 2% of the MMA as initially used) was collected for dewatering.

1.31 g (0.19 wt% or 0.23 mol%, based on the amount of MA-2 used, 1:10 dissolved in MeOH) of lithium methoxide was entered to the reaction mixture.

During reaction time a permanent fresh MMA feed of 1.5 mL/min was maintained until the overall ratio of MMA:MA-2 = 20.7:1 was reached.

In steps of 1 hour, the distillate was distilled off in portions to the outside of the reaction system until temperature in the column head reached 100°C again every time. After 4 times distillate take off the reactor was filled with air to ambient pressure and the heating was stopped.

At a temperature of 100°C, 4 g of the filtration aid Arbocel^{®} FIC 200 (0.5 wt%, based on the total weight of MM-2) was added and the mixture stirred for 30 minutes until reaching 70°C.

The resulting reaction mixture was filtered at a temperature of 70°C using a SEITZ-T 1000 filter. The reaction mixture was further heated up to 130°C and stirred under vacuum of 10 mbar for 1-2 hours to remove excess MMA completely.

The final reaction mixture was filtered again at a temperature of 70°C using a SEITZ-T 1000 filter.

The yield (based on the raw material alcohol) was 99.9%.

### Example 12: MMA:MA-2 = 25.9 :1 reaction under vacuum

A reaction vessel equipped with a stirrer, a thermometer, a reflux condenser and a gas introduction apparatus was charged with 1000 g (0.15 mol) of macroalcohol MA-2 (70% in NB3020), 388.9 g (3.88 mol) of MMA, 0.078 g (200 ppm, based on the amount of MMA used) of hydrochinone monomethylether, 0.019 g (50 ppm, based on the amount of MMA used) of Tempol.

After charging the raw materials and start of stirring and air bubbling with 20 L/h, the reaction mixture was heated to reflux.

1.39 g (0.2 wt% or 0.24 mol%, based on the amount of MA-2 used) of lithium methoxide was entered to the reaction mixture and a slight vacuum of 900 bar was maintained during the reaction time.

In steps of 1 hour, the distillate was distilled off in portions to the outside of the reaction system until temperature in the column head reached 100°C again every time. After 4 times distillate take off the reaction mixture was stirred at 130°C under vacuum of 10 mbar for 1-2 hours to remove excess MMA completely.

The reactor was filled with air to ambient pressure and the heating was stopped.

At a temperature of 100°C, 5 g of the filtration aid Arbocel^{®} FIC 200 (0.5 wt%, based on the total weight of MM-2) was added and the mixture stirred for 30 minutes until reaching 70°C.

The resulting reaction mixture was filtered at a temperature of 70°C using a SEITZ-T 1000 filter.

The yield (based on the raw material alcohol) was 0%.

### Example 13: MMA:MA-1 = 22.7:1

A reaction vessel equipped with a stirrer, a thermometer, distillation column, a reflux condenser and a gas introduction apparatus was charged with 4033 kg (627.3 mol) of macroalcohol MA-2 (70% in NB3020), 1424 kg (14240 mol) of MMA, 400 g (100 ppm, based on the amount of MMA used) of hydrochinone monomethylether.

After charging the raw materials stirring and air bubbling with 8000 L/h were started as well as stabilizer dosing of 5 L/h (3000 ppm MEHQ in MMA) into the column head. The reaction mixture was then heated to reflux and 100% distillate taken off until 101 L distillate (corresponding to about 6% of the MMA as initially used) was collected for dewatering.

6 kg (0.21 wt% or 0.25 mol%, based on the amount of MA-2 used) of lithium methoxide was entered to the reaction mixture within 2 portions; 4 kg after dewatering step and 2 kg after 4 hours of reaction time. In steps of 1 hour, the distillate was distilled off in portions to the outside of the reaction system until temperature in the column head reached 100°C again every time. After 4 times distillate take off the reaction mixture was further heated up to 142°C and stirred under vacuum of 53 mbar for 45 minutes to remove excess MMA completely.

The reactor was filled with air to ambient pressure and the heating was stopped.

At a temperature of 90°C, 12 kg of the filtration aid Arbocel^{®} FIC 200 (0.25 wt%, based on the total weight of MM-2) was added and the mixture stirred for 120 minutes at 90-100°C.

The resulting reaction mixture was filtered at a temperature of 70°C using a rotating disc filter while in the first 22 min the material was circulated over the filter before switching to throughput filtration.

The yield (based on the raw material alcohol) was 99.9%.

The following Table 2 gives an overview of the conversion received for the macromonomers prepared.

**Table 2: Overview of the conversion received for the macromonomers prepared.**

| **Example #** | **Ratio of MMA : MA** | **Molar ratio catalyst: MA** | **conversion** | **Purity MA-1/2** | **Dewatering before catalyst intake** | **Remarks** |
|---|---|---|---|---|---|---|
| 1 | 15:1 | 0.14 | 100% | 100% | Y | |
| 2 | 15:1 | 0.17 | >95% | 100% | N | |
| 3 | 15:1 | 0.095 | >95% | 100% | N | |
| 4 | 10:1 | 0.08 | 100% | 100% | N | |
| 5 | 6:1 | 0.087 | 99.5% | 100% | N | |
| 6 | 8:1 | 0.087 | 99.5% | 70% | N | |
| 7 | 5:1 | 0.085 | 96.4% | 70% | N | |
| 8 | 8:1 | 0.125 | 99.8% | 100% | Y | |
| 9 | 22:1 | 0.32 | 100% | 70% | Y | |
| 10 | 28.5:1 | 0.25 | 100% | 70% | N | |
| 11 | 20.7:1 | 0.23 | 99.9% | 70% | Y | |
| 12 | 25.9:1 | 0.24 | 0% | 70% | N | reaction under vacuum |
| 13 | 22.7:1 | 0.25 | 99.9% | 70% | Y | |

## Claims

1. Process for preparing a high molecular weight (meth)acrylic acid ester by transesterification of a C1-4 alkyl (meth)acrylate with a macroalcohol in the presence of a catalyst, wherein the molar ratio of the C1-4 alkyl (meth)acrylate : macroalcohol in the reaction mixture is between 5:1 and 50:1.

2. The process according to claim 1, **characterized in that** the macroalcohol has a number-average molecular weight Mₙ in the range of 800 g/mol to 8000 g/mol.

3. The process according to claim 1, **characterized in that** the macroalcohol has a number-average molecular weight Mₙ in the range of 1000 g/mol to 5000 g/mol.

4. The process according to claim 1, **characterized in that** the transesterification catalyst is used in an amount of 0.01 wt% to 0.8 wt%, preferably 0.1 wt% to 0.3 wt%, based on the weight of the macroalcohol used, and is selected from the group consisting of tetraisopropyltitanate, ethylhexyltitanate, tetralauryltitanate, lithium methoxide, sodium methoxide, calcium hydroxide, magnesium hydroxide and mixtures thereof.

5. The process according to claim 4, **characterized in that** the transesterification catalyst is lithium methoxide, used in solid form or dissolved in methyl methacrylate or methanol as 10% solution.

6. The process according to claim 1, **characterized in that** the reaction mixture further comprises a polymerization inhibitor which is selected from the group consisting of hydrochinone monomethylether, 4-hydroxy-2,2,6,6-tetramethylpiperidinooxyl, octadecyl-3-(3,5-di-tert-butyl-4-hydroxyphenyl)-propionate, 4-methyl-2,6-di-tert-butylphenol and mixtures thereof, whereby a permanent air stream of 5 L/h to 80 L/h (corresponding to 6 vol% to 21 vol% of oxygen) through the reaction mixture is maintained.

7. The process according to claim 1, **characterized in that** the transesterification reaction is performed at a temperature between 70°C and 155°C.

8. The process according to claim 1, **characterized in that** the molar ratio of the C1-4 alkyl (meth)acrylate : macroalcohol in the reaction mixture is between 5:1 and 30:1.

9. The process according to claim 1, **characterized in that** the by-produced C1-4 alkyl (meth)acrylate/C1-4 alcohol azeotrope is removed from the reaction system by batch or continuous distillation.

10. The process according to claim 1, **characterized in that** the used catalyst is subsequently removed by using functional cellulose fibers or calcined diatomaceous earth as filtration aids.

11. The process according to claim 10, **characterized in that** the filtration aid is used in an amount of 0.01 wt% to 3 wt%, based on the total weight of the (meth)acrylic acid ester.

12. Process for preparing a high molecular weight (meth)acrylic acid ester by transesterification of a C1-4 alkyl (meth)acrylate with a macroalcohol, comprising the steps of:
(a) charging a reaction vessel equipped with stirrer and reflux condenser with a C1-4 alkyl (meth)acrylate and a macroalcohol in a ratio of between 5:1 and 50:1 and at least one stabilizer;
(b) maintaining a permanent air stream of 5 L/h to 80 L/h (corresponding to 6 vol% to 21 vol% of oxygen) through the reaction mixture;
(c) optionally, heating the reaction mixture obtained under (b) until a stable reflux is reached and distilling about 1% to 10% of the C1-4 alkyl (meth)acrylate to remove water from the reaction system;
(d) adding 0.1 wt% to 0.3 wt% of a catalyst, based on the total amount of macroalcohol used, in solid form or as a 10% solution in methyl methacrylate or methanol;
(e) heating the reaction mixture obtained under (d) until the head temperature of the condenser reaches reflux temperature of the lower chain alkyl (meth)acrylate used or its azeotrope with the released low molecular alcohol;
(f1) constantly distilling an azeotropic mixture of the C1-4 alkyl (meth)acrylate and the corresponding C1-4 alkyl alcohol which is built during transesterification until the head temperature of the condenser reaches the temperature of the pure C1-4 alkyl (meth)acrylate used; or
(f2) holding the reaction mixture obtained under (e) under total reflux to enrich the corresponding C1-4 alkyl alcohol which is built during transesterification in the azeotrope and taking off the azeotrope in certain intervals;
(g) after the corresponding C1-4 alkyl alcohol is completely removed, removing of the excessive C1-4 alkyl (meth)acrylate under reduced pressure during a time of 1 to 2 hours;
(h) filling the reaction vessel with air or nitrogen to ambient pressure and cooling down the reaction mixture obtained under (g) to a temperature in the range of 80°C to 100°C;
(i) adding 0.1 wt% to 3 wt% of a filtration aid, based on the total weight of the high molecular weight (meth)acrylic acid ester, and mixing; and
(j) cooling down the mixture obtained under (i) to a temperature of about 70°C and filtrating it by using a filter to receive the high molecular weight (meth)acrylic acid ester.

13. Process for preparing a high molecular weight (meth)acrylic acid ester by transesterification of methyl (meth)acrylate with a macroalcohol, comprising the steps of:
(a) charging a reaction vessel equipped with stirrer and reflux condenser with methyl methacrylate and a macroalcohol in a ratio of between 5:1 and 30:1, the macroalcohol having a number-average molecular weight Mₙ of 1000 g/mol to 5000 g/mol, and at least one stabilizer which is selected from the group consisting of hydrochinone monomethylether, 4-hydroxy-2,2,6,6-tetramethylpiperidinooxyl, octadecyl-3-(3,5-di-tert-butyl-4-hydroxyphenyl)-propionate, 4-methyl-2,6-di-tert-butylphenyl and mixtures thereof;
(b) maintaining a permanent air stream of 5 L/h to 80 L/h (corresponding to 6 vol% to 21 vol% of oxygen) through the reaction mixture;
(c) optionally, heating the reaction mixture obtained under (b) until a stable reflux is reached and distilling about 1% to 10% of the methyl methacrylate to remove water from the reaction system;
(d) adding 0.1 wt% to 0.3 wt% of lithium methoxide, based on the total weight of the reaction mixture, as solid catalyst;
(e) heating the reaction mixture obtained under (d) until the head temperature of the condenser reaches reflux temperature of 65°C to 100°C;
(f1) constantly distilling off an azeotropic mixture of methyl methacrylate and methanol which is built during transesterification until the head temperature of the condenser reaches a temperature of about 95°C to 100°C; or
(f2) holding the reaction mixture obtained under (e) under total reflux to enrich the methanol which is built during transesterification in the azeotrope and taking off the azeotrope from the head of the distillation column after certain intervals;
(g) after methanol is completely removed, removing the excessive methyl methacrylate under reduced pressure during a time of 1 to 2 hours;
(h) filling the reaction vessel with air or nitrogen to ambient pressure and cooling down the reaction mixture obtained under (g) to a temperature of about 80°C to 100°C;
(i) adding 0.1 wt% to 3 wt% of a filtration aid, based on the total weight of the high molecular weight (meth)acrylic acid ester, and mixing; and
(j) cooling down the mixture obtained under (i) to a temperature of about 70°C and filtrating it by using a filter to receive the macromonomer.

14. Use of the macromonomers obtained by the process according to any one of claims 1 to 13 for the preparation of polymers or copolymers.

15. Use of the macromonomers obtained by the process according to any one of claims 1 to 13 for preparing polymers or copolymers which can be used as additives in lubricant compositions, e.g. as viscosity index improver, antioxidants, antiwear agents, dispersants, detergents, friction modifiers, antifoam agents, extreme pressure additives, corrosion inhibitors, pour point depressants and fluids for thermal management.
